# FASCICULE DE BREVET EUROPEEN

(11) **EP 0 711 129 B1**
(45) Date de publication et mention de la délivrance du brevet: **16.06.1999**
(21) Numéro de dépôt: 93924114.7
(22) Date de dépôt: 27.10.1993
(51) Int. Cl.: A61B 17/34

(54) **TROCART A PROTECTION DE POINTE**
TROKAR MIT GESCHÜTZTER SPITZE
TROCAR PROVIDED WITH A PROTECTED TIP

(30) Priorité: 28.10.1992 FR 9212841
(43) Date de publication de la demande: 15.05.1996
(73) Titulaire: MELIN, Cyriac, F-52800 Nogent (FR)
(72) Inventeur: MELIN, Cyriac, F-52800 Nogent (FR)
(74) Mandataire: Bruder, Michel
(86) Numéro de dépôt international: FR9301051
(87) Numéro de publication internationale: WO9409712

(56) Documents cités:
- WO-A-92/10141
- DE-U- 9 112 976
- US-A- 1 882 213
- US-A- 4 535 773

## Description

La présente invention concerne un trocart à protection de pointe. Ce trocart est plus particulièrement destiné à la coelioscopie et aux opérations endoscopiques du même genre.

On connaît déjà des trocarts pour la coelioscopie intestinale, vésiculaire ou gynécologique qui sont pourvus de dispositifs de protection de la pointe servant à perforer, en début d'opération, l'enveloppe musculaire ou tissulaire des organes à visualiser ou à opérer. En particulier, il existe des tubes qui sont montés coulissant et de manière élastique à l'extérieur du trocart pour venir entourer la pointe dès qu'elle a perforée l'enveloppe anatomique, par exemple le péritoine dans le cas d'une opération vésiculaire. C'est le cas par exemple des trocarts décrits dans le brevet US-4.535.773 qui sont constitués d'un premier tube creux extérieur dans lequel vient s'engager un dispositif de perforation de la membrane anatomique dont l'extrémité distale est constituée d'une pointe polyédrique, ledit dispositif coulissant juste à l'intérieur d'un deuxième tube creux, monté libre coaxialement à l'intérieur du premier et muni de moyens élastiques pour venir recouvrir la pointe polyédrique dès que celle-ci a perforé la membrane anatomique.

Malgré la performance de ce type de trocart à protection de pointe, on constate néanmoins quelques accidents, le plus souvent graves, qui se produisent essentiellement pour les deux raisons suivantes :
- l'enveloppe anatomique, par exemple le péritoine, présente une élasticité telle que la pointe du trocart provoque son ouverture d'une manière relativement brusque ; de ce fait, l'introduction complète du tube de protection extérieur dans le passage ménagé par la pointe du trocart se produit avec un temps de retard qui risque d'être d'autant plus dommageable pour les organes et les tissus internes que le mouvement du praticien est plus vif. Ne remplissant donc pas complètement son rôle au moment de la perforation, le tube de protection devient même indirectement dangereux puisqu'il est en effet difficile au praticien de se rendre compte que la pointe du trocart n'a pas été protégée immédiatement après le franchissement de la membrane anatomique.
- en outre, même dans le cas où le tube de protection remplit correctement sa fonction, il n'en demeure pas moins que le bord distal de ce tube est relativement tranchant, ce qui vient parfois blesser les tissus internes qu'il est censé prémunir ; le tube devient alors aussi dangereux que la pointe elle-même.

La présente invention vise à remédier à ces inconvénients en proposant un trocart destiné à la coelioscopie et aux opérations analogues sur les êtres vivants, comprenant un tube extérieur creux protégeant les tissus et les organes à visualiser ou à opérer dans lequel vient exactement s'engager un dispositif de perforation de la membrane anatomique dont l'extrémité distale est une pointe polyédrique, caractérisé en ce que ledit dispositif de perforation est formé par un tube creux dont l'extrémité distale est constituée de facettes évidées permettant soit une visualisation directe du trajet de la pointe par le moyen d'un endoscope engagé à cet effet par la partie proximale dudit dispositif de perforation, soit le passage d'un mécanisme de protection de pointe actionné coaxialement et de manière élastique en direction de ladite pointe au travers d'au moins une des facettes de cette dernière.

Dans ses deux modes d'utilisation, le trocart conforme à l'invention est beaucoup plus sûr que les trocarts connus jusque là.

Muni d'un endoscope, le trocart se conduit au travers des tissus d'une manière parfaitement contrôlée. A cet égard, même s'il advient qu'une ou plusieurs facettes de la pointe soit encombrée par exemple par de la graisse, il est rare que la visibilité de l'endoscope soit réduite à néant ; en outre, les endoscopes généralement utilisés avec les trocarts sont pourvus de moyens de désembuage qui contribuent bien sûr à améliorer la vision du praticien.

Selon une autre variante utilisable en coelioscopie, le trocart conforme à l'invention est sans doute encore plus sûr. En effet, un mécanisme de protection de la pointe agissant par l'intérieur du dispositif de perforation entre plus vite en action qu'un tube extérieur au passage d'une membrane et, d'un autre côté, les inconvénients liés au risque de blessure dû au bord distal tranchant d'un tel tube disparaissent totalement. En outre, alors que pour cette même dernière raison, il n'était guère possible de réaliser un tube de protection extérieur en métal ou en acier stérilisable, le mécanisme de protection interne tel que proposé par l'invention est normalement fabriqué en acier inoxydable et peut donc être utilisé aussi souvent que nécessaire.

D'autres caractéristiques et avantages de la présente invention ressortiront mieux de la description qui va suivre d'un mode de réalisation préférentiel d'un trocart muni d'un dispositif de perforation creux à pointe polyédrique et facettes évidées, donné à titre d'exemple non limitatif en référence aux dessins annexés sur lesquels :
- la figure 1 est une vue de montage éclatée du trocart, montrant ses pièces constitutives principales telles qu'elles peuvent être séparément stérilisées,
- les figures 2 à 5 sont des vues latérales du trocart successivement à l'état de repos (pointe protégée), à l'état d'armement du mécanisme de protection (juste avant la perforation de la membrane), à l'état d'effacement dudit mécanisme de protection (pendant la perforation) et à l'état de retour de ce mécanisme à l'état de repos (juste après perforation),
- les figures 6, 7 et 8 sont des vues latérales partielles d'une seconde variante de la tête pour la protection de la pointe du trocart selon l'invention, représentant à plus grande échelle que celle des figures 2 à 5, les états successifs d'actionnement de ladite protection de pointe.
- la figure 9 est une vue extérieure de l'ensemble du trocart monté, équipé de la seconde variante de protection de pointe telle que représentée aux figures 6 à 8.

Le trocart qui sera décrit maintenant est plus particulièrement destiné à la coelioscopie. Il comporte un tube extérieur creux 1 (figure 9) dans lequel vient exactement s'engager un dispositif de perforation 2 servant au perçage du péritoine du patient. Dans ce type d'opération, l'introduction du tube extérieur creux 1 du trocart au travers du péritoine a pour objectif de former ce que les praticiens nomment un pneumopéritoine, opération qui consiste à insuffler un gaz neutre et stérile sous pression dans la poche péritonique ; à cet effet, le tube extérieur 1, par lequel on peut prévoir d'introduire le gaz en question, est pourvu d'une valve centrale à actionnement mécanique telle qu'il est par exemple décrit dans le brevet FR-A-2.689.384 ou dans le document correspondant EP-A-0.564.373 ; le tube extérieur 1 comporte également un moyen annexe de mise en dépression du pneumopéritoine et des moyens de centrage 1a des instruments coelioscopiques qui peuvent y être introduits.

Suivant la présente invention, le dispositif de perforation 2 est formé d'un tube creux proximal 3 ouvert à ses deux extrémités, se terminant en partie distale par une pointe polyédrique 4 dont les facettes 4a sont évidées et dont seules les arêtes 4b sont ainsi présentes. L'effilage de ces dernières et la finesse du sommet 4c de la pointe 4 sont en tout point comparables à ceux de la pointe pleine d'un dispositif de perforation conventionnel.

Selon une première utilisation de l'invention, un endoscope, non représenté sur les figures, est introduit par l'orifice proximal du tube creux 3 jusqu'à venir pratiquement en butée dans le fond évidé de la pointe 4, ce qui permet au praticien de visualiser exactement le passage du trocart au travers des tissus. En outre, il est possible d'agencer classiquement un tube de protection autour du dispositif de perforation 2 ainsi constitué, en associant donc ainsi une double sécurité mécanique et visuelle.

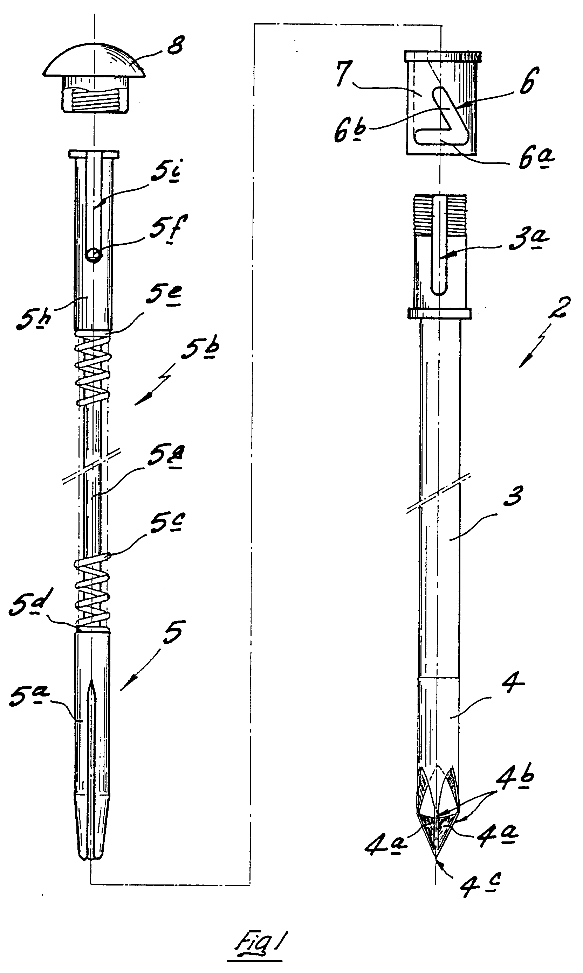

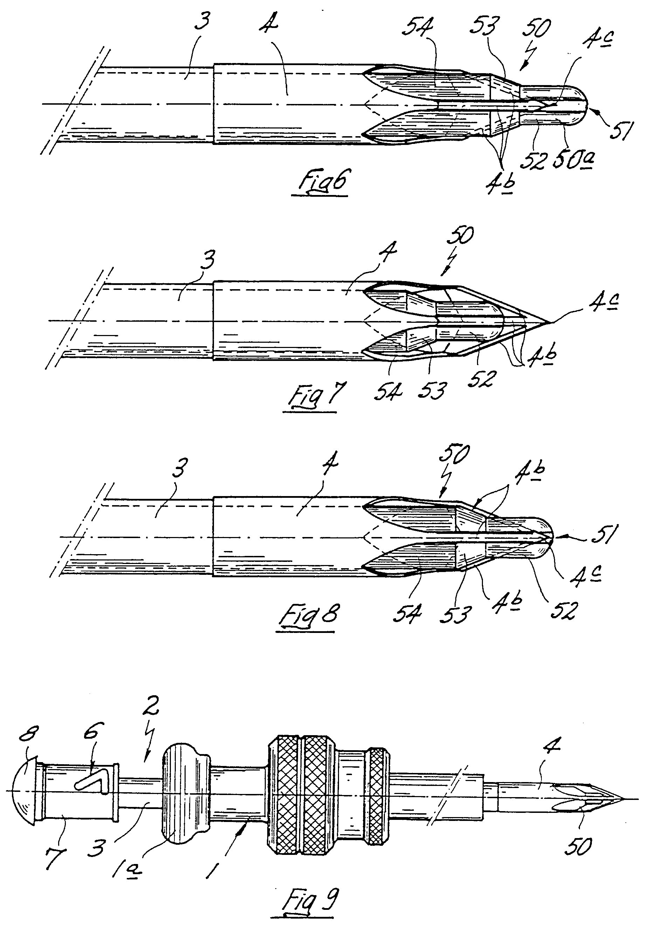

Selon une seconde utilisation de l'invention telle que représentée plus en détail sur les figures 1 à 5, un mécanisme de protection de pointe 5 est introduit coaxialement dans le tube creux 3 du dispositif de perforation 2 de manière à prémunir mécaniquement les tissus et les organes de tout risque de blessure. A cet effet, ce mécanisme de protection de pointe 5 est constitué par l'assemblage, d'avant en arrière du trocart :
- d'une tête distale 5a en forme d'obus à plusieurs secteurs longitudinaux, dont le rôle est de venir coulisser le long de la pointe 4, autour des arêtes 4b, dans les espaces laissés libres par les facettes évidées 4a ;
- d'un ensemble d'actionnement 5b sollicitant élastiquement ladite tête 5a vers l'avant du dispositif de perforation 2 de manière à dégager la pointe 4 seulement lorsqu'une force de recul suffisante est appliquée sur la tête 5a, ce qui est notamment le cas lors de la perforation du péritoine ; un réglage correct du seuil au-delà duquel l'ensemble d'actionnement 5b autorise le dégagement de la pointe 4 procure ainsi un moyen très simple pour s'assurer qu'au moment de la rupture du péritoine, le mécanisme de protection de pointe 5 sera très vite et automatiquement mis en action.

En outre, les longueurs respectives du mécanisme de protection de pointe 5 et du tube creux 3 sont prévues pour qu'en position de repos, la tête distale 5a dudit mécanisme 5 vienne entourer complètement la pointe 4. Par ailleurs, il est prévu que l'extrémité distale arrondie de l'obus formé par ladite tête 5a dépasse de quelques millimètres l'extrémité 4c de ladite pointe 4 ; cette disposition relative de l'extrémité 4c de la pointe et de la tête 5a permet de faire en sorte que le mécanisme de protection de pointe 5 soit armé - c'est-à-dire ait tendance à revenir à sa position initiale - avant que l'extrémité 4c n'émerge de la tête 5a.

Un exemple particulier et non limitatif de réalisation du mécanisme d'actionnement 5b de la tête 5a du mécanisme de protection de pointe 5 sera maintenant décrit plus en détail en référence à la figure 1.

L'ensemble d'actionnement 5b de la tête 5a comprend notamment un ressort 5c, maintenu comprimé entre une butée distale 5d juste située à l'arrière de la tête 5a et une butée proximale 5e, la butée distale 5d pouvant être déplacée le long du dispositif de perforation 2 pour venir encore comprimer ledit ressort 5c. Le déplacement longitudinal de la tête 5a, à l'encontre de la force d'appui exercée par le ressort 5c contre la butée distale 5d, est contrôlé par le moyen d'un doigt radial 5f qui est engagé à cet effet dans un chemin de came 6 d'une double douille 7 fixée à l'arrière du tube creux 3 dudit dispositif de perforation 2 par un écrou vissé 8. Le doigt radial 5f, constitué à la manière d'un ergot élastique effaçable radialement, est disposé d'une manière non limitative à l'extrémité proximale d'une tige de liaison 5g, fixée à son autre extrémité à la tête distale 5a ; cette tige de liaison 5g s'étend longitudinalement et coaxialement au milieu du ressort 5c puis traverse la butée proximale 5e pour venir finalement émerger coaxialement dans un organe de guidage 5h, lequel est pourvu d'une saignée longitudinale 5i au milieu de laquelle est susceptible de se déplacer le doigt radial 5f. Dans cette disposition de l'invention, le bord inférieur de l'organe de guidage 5h joue bien sûr le rôle de la butée proximale 5e.

Le chemin de came 6 de la double douille 7, qui est monté librement autour de l'organe de guidage 5h, est formé d'un premier segment de droite transversal 6a relié, à l'une de ses extrémités, à un second segment de droite 6b qui est incliné par rapport à la direction longitudinale du trocart et dirigé vers l'arrière de ce dernier par rapport audit premier segment 6a ; les mouvements relatifs de la douille 7 et de l'organe de guidage 5h sont limités par les déplacements du doigt radial 5f dans le chemin de guidage 6, ledit doigt radial 5f étant lui-même introduit, au moment du montage, dans une saignée longitudinale 3a prévue à cet effet à l'extrémité proximale du tube 3 du dispositif de perforation 2 ; cette saignée 3a sert de détrompeur angulaire pour l'introduction correcte du dispositif de protection de pointe 5 au milieu du tube 3, et permet notamment de faire en sorte que chaque secteur angulaire de la tête distale 5a en forme d'obus vienne s'introduire sans difficulté au milieu des facettes évidées 4a de la pointe 4.

Suivant une autre caractéristique de l'invention, la douille 7 présente une face interne de forme tronconique dont la pente est prévue pour que, sous la sollicitation du ressort 5c, le doigt radial 5f soit ramené le plus directement possible en direction du premier segment 6a ; ainsi, lorsque ledit doigt radial 5f est situé à une hauteur quelconque le long du second segment 6b et qu'aucune force de recul n'est appliquée sur la tête distale 5a du mécanisme de protection 5 pour dégager la pointe 4, le doigt radial 5f peut s'escamoter rapidement vers l'intérieur de la saignée longitudinale 5i de l'organe de guidage 5h de manière à glisser sur la face interne inclinée de la douille 7 jusqu'à ce qu'il puisse à nouveau émerger, d'une manière élastique, dans l'ouverture du premier segment 6a du chemin de came 6 ; le doigt radial 5f est alors bloqué longitudinalement dans ledit segment 6a, interdisant de ce fait tout dégagement de la pointe 4 hors de la tête distale 5a. Cette dernière disposition de l'invention permet de faire en sorte que le mécanisme de protection de pointe 5 soit activement sollicité vers l'avant, c'est-à-dire armé, en permanence, y compris dans la position où il recouvre complètement la pointe 4, ce qui procure bien sûr une sécurité accrue.

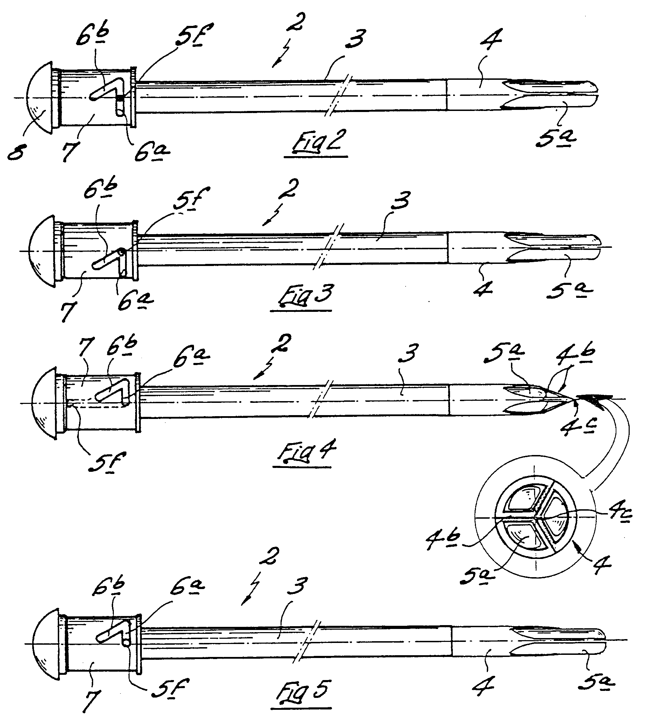

Le fonctionnement du mécanisme de protection de pointe 5 sera maintenant mieux décrit en référence aux figures 2 à 5 :
- afin de mettre le dispositif de perforation 2 dans la position de repos représentée sur la figure 2, le praticien enclenche le doigt radial 5f du mécanisme de protection de pointe 5 le long du segment transversal 6a du chemin de came 6 de la double douille 7, ce qui bloque tout mouvement relatif du tube creux 3 et du mécanisme 5 et donc tout dégagement de la pointe 4.
- juste avant de venir perforer le péritoine du patient, le praticien fait pivoter la douille 7 sur elle-même de manière à amener le doigt radial 5f au point d'intersection des deux segments 6a et 6b du chemin de came 6, dans la position dite d'armement du mécanisme de protection de pointe 5, représentée sur la figure 3. En effet, dans cette situation, le doigt radial 5f est susceptible de reculer le long du segment incliné 6b dès qu'une force suffisante sera exercée sur la tête distale 5a dudit mécanisme 5 pour dégager au moins partiellement la pointe 4.
- cette situation se produit dès que le praticien applique l'extrémité distale du mécanisme de protection 5 contre le péritoine, ce qui provoque d'abord le recul de la tête 5a puis son effacement partiel, laissant émerger le sommet 4c et les arêtes 4b de la pointe 4. Cet état d'effacement dudit mécanisme de protection 5 est représenté sur la figure 4 dont un détail agrandi montre, vue de face, la position relative de la tête 5a et de la pointe 4.
- juste après la perforation du péritoine, qui se produit d'une manière relativement brusque, pratiquement plus aucune force n'agit pour retenir la tête distale 5a du mécanisme de protection 5, toujours sollicitée vers l'avant du trocart par son mécanisme d'actionnement 5b, ce qui permet à ladite tête 5a de revenir très rapidement autour de la pointe 4. On comprend mieux ici l'intérêt procuré par l'agencement du plan incliné tronconique à l'intérieur de la douille 7 - qui permet au doigt radial 5f de s'escamoter directement sous la douille 7, sans avoir à suivre le segment incliné 6b du chemin de came 6 -. En l'absence de cette disposition particulière de l'invention, le retour du mécanisme de protection de pointe 5 vers l'avant du trocart nécessiterait une rotation de ladite douille 7 sur elle-même, avec un risque toujours possible de blocage du doigt radial 5f le long du chemin de guidage 6.

En référence aux figures 6 à 8 des dessins, on décrira ci-après une autre variante du mécanisme de protection de pointe 50. Dans cette variante, il a été surtout recherché une forme de tête du mécanisme augmentant sensiblement sa rapidité de rappel, dès lors que la perforation tissulaire est suffisante pour l'introduction du trocart ; on sait qu'une telle perforation s'accompagne souvent de blessures des organes environnants sous le tissu perforé, dont la gravité dépend, bien entendu, de la proximité de ces organes par rapport audit tissu.

Dans cette variante il a donc été élaboré un dispositif de protection étagé permettant d'effectuer la perforation en deux temps : on perfore d'abord le tissu, par exemple le péritoine, grâce à l'extrémité 4c de la pointe 4 du trocart, ce qui déclenche quasi immédiatement une première avancée du mécanisme de protection conformé pour venir recouvrir totalement l'extrémité 4c et éviter ainsi de blesser les organes environnants sous le tissu ainsi perforé. Dans un deuxième temps, l'extrémité 4c de la pointe 4 étant protégée, ce sont ses arêtes tranchantes 4b qui poursuivent la perforation jusqu'au diamètre souhaité le mécanisme de protection 50 étant toujours maintenu en positon de recul ; dès que le diamètre souhaité est obtenu, ledit mécanisme est alors brutalement rappelé dans le sens de l'avance du trocart venant ainsi définitivement recouvrir les arêtes tranchantes 4b de la pointe 4, mettant définitivement à l'abri tous les organes menacés.

A cet effet, le mécanisme de protection de pointe 50 est constitué par l'assemblage suivant :
- un ensemble d'actionnement (non représenté sur les figures 6 à 8) en tout point identique à celui qui a été décrit dans la première variante,
- une tête distale 50a en forme d'obus étagé comportant comme dans la première variante, plusieurs secteurs longitudinaux susceptibles de venir coulisser le long de la pointe polyédrique 4 autour de ses arêtes 4b au travers des espaces laissés libres par les facettes évidées 4a de la pointe 4.

La tête distale 50a du mécanisme de protection 50 diffère de la première variante en ce qu'elle comprend au moins deux étages 52,54 raccordés par une zone tronconique 53, la partie étagée 52 la plus fine étant située à l'extrémité de la tête afin de limiter la résistance à la pénétration dudit mécanisme au travers du tissu et diminuer ainsi les temps de réaction pour la couverture de la pointe 4. La finesse de la partie extrême 52 n'est seulement limitée qu'en considération du danger que pourrait représenter un dispositif de couverture trop effilé. La longueur de ladite partie extrême 52 n'est, quant à elle, dictée que par un compromis entre la rapidité du rappel de la protection et la facilité de la perforation que l'on souhaite réaliser ; à cet égard, la longueur de la partie extrême 52 est telle que lorsqu'elle recouvre exactement l'extrémité 4c de la pointe 4 du trocart, il reste une longueur de coupe suffisante procurant l'action des seules arêtes tranchantes 4b du trocart qui viennent terminer la perforation avant recouvrement complet desdits tranchants 4b par rappel élastique du deuxième étage 54 de section légèrement inférieure à la section interne du tube 3 du trocart, selon un processus déjà détaillé dans la variante précédente. Enfin, la partie extrême 52 de la tête 50a est réunie à l'étage 54, par une zone de raccordement tronconique 53 dont l'angle d'inclinaison est avantageusement identique à celle des arêtes tranchantes 4b de la pointe 4 du trocart par rapport à son axe longitudinal.

Selon cette seconde variante, le praticien utilise le trocart de la manière suivante :
- une première approche est effectuée sur la zone du péritoine retenue pour la perforation, l'extrémité 4c du trocart et ses arêtes tranchantes 4b étant complètement recouvertes par le mécanisme de protection 50, l'outil reposant sur la pointe arrondie 51 du mécanisme de protection. Par une action d'arrière en avant, le mécanisme de protection (figure 6) a tendance à reculer, conformément à la figure 7, permettant alors le dégagement progressif de l'extrémité 4c du trocart qui peut alors effectuer une perforation préliminaire du tissu. On a vu que l'élargissement de la perforation s'effectuait ensuite au moyen des arêtes tranchantes 4b de la pointe polyédrique du trocart. Dès que le diamètre de la perforation préliminaire est suffisant, la partie affinée de l'étage extrême 52 du mécanisme 50 est alors à même de pénétrer dans cette perforation préliminaire et venir ainsi très tôt et très rapidement couvrir l'extrémité 4c du trocart puisqu'il n'est pas nécessaire d'attendre que la perforation ait atteint son diamètre final ; le mécanisme du trocart est alors dans la disposition de la figure 8 et seuls les arêtes tranchantes 4b de la pointe 4 sont maintenant au contact de la perforation préliminaire permettant ainsi de l'élargir comme souhaité ; avant élargissement, la tête 50a du mécanisme qui se trouve en appui sur la zone inclinée 53 tend à maintenir le ressort de rappel en position d'armement jusqu'à ce que l'élargissement de la perforation ait atteint le diamètre voulu ouvrant ainsi le passage pour la partie étagée 54 du mécanisme de protection 50 qui vient définitivement recouvrir l'ensemble des éléments coupants du trocart ; ce dernier se retrouve alors dans la position de la figure 6, c'est-à-dire dans sa position de repos.

Bien entendu, l'invention n'est pas limitée par les exemples qui viennent d'en être donnés mais s'étend à toutes les variantes de réalisation analogues prévoyant un dispositif de protection de la pointe d'un dispositif de perforation pour trocart agissant colinéairement et par le milieu de ladite pointe. En particulier, l'invention n'est pas limitée à l'utilisation d'une pointe 4 triédrique ; on pourra également faire en sorte que la tête du mécanisme de protection 5 ou 50 ne vienne pas entourer la totalité de ses arêtes 4b, mais ne traverse qu'une seule de ses facettes 4a ; à cet égard, il est clair qu'il n'est pas non plus nécessaire d'évider la totalité des mêmes facettes 4a pour mettre en oeuvre l'invention.

## Revendications

1. Trocart destiné à la coelioscopie et aux opérations analogues sur les êtres vivants, comprenant un tube extérieur creux (1) protégeant les tissus et les organes à visualiser ou à opérer, dans lequel vient exactement s'engager un dispositif de perforation (2) de la membrane anatomique dont l'extrémité distale est une pointe polyédrique caractérisé en ce que ledit dispositif de perforation (2) est formé par un tube creux (3) dont l'extrémité distale est constituée de facettes (4a) évidées permettant soit une visualisation directe du trajet de la pointe (4) par le moyen d'un endoscope engagé à cet effet par la partie proximale dudit dispositif de perforation (2), soit le passage d'un mécanisme de protection de pointe (5,50) actionné coaxialement et de manière élastique en direction de ladite pointe (4), au travers d'au moins une des facettes (4a) de cette dernière.

2. Trocart selon la revendication précédente, caractérisé en ce que le mécanisme de protection de pointe (5,50) est constitué par l'assemblage d'une tête distale (5a,50a) en forme d'obus à plusieurs secteurs longitudinaux, dont le rôle est de venir coulisser le long des arêtes (4b) de la pointe (4), et d'un ensemble d'actionnement (5b) sollicitant élastiquement ladite tête distale (5a,50a) vers l'avant du dispositif de perforation (2) de manière à dégager la pointe (4) seulement lorsqu'une force de recul suffisante est appliquée sur ladite tête (5a,50a).

3. Trocart selon la revendication 2, caractérisé en ce que la tête (50a) du mécanisme de protection de pointe (50) a la forme d'un obus étagé comprenant au moins une partie extrême (52) effilée et un second étage (54) de section légèrement inférieure à la section interne du tube (3) du trocart, raccordés entre eux par une zone (53) de forme tronconique.

4. Trocart selon la revendication 3, caractérisé en ce que la section de la partie extrême (52) est la plus faible possible ne risquant pas de blesser les organes après le début de la perforation, la longueur de ladite partie (52) étant suffisante pour que, lorsque l'extrémité 4c de la pointe 4 est juste recouverte par ladite partie (52) les arêtes tranchantes (4b) de la pointe (4) restent suffisamment en saillie pour terminer la perforation.

5. Trocart selon l'une quelconque des revendications 3 ou 4, caractérisé en ce que la zone (53) de raccordement présente une inclinaison par rapport à l'axe longitudinal du mécanisme de protection de pointe (50) identique à l'inclinaison des arêtes 4b de la pointe 4.

6. Trocart selon l'une quelconque des revendications précédentes, caractérisé en ce que les longueurs respectives du mécanisme de protection de pointe (5,50) et du tube creux (3) du dispositif de perforation (2) sont prévues pour qu'en position de repos, la tête distale (5a,50a) dudit mécanisme (5,50) vienne entourer complètement la pointe (4).

7. Trocart selon la revendication précédente, caractérisé en ce que l'extrémité distale arrondie de l'obus formé par ladite tête (5a,50a) dépasse de quelques millimètres l'extrémité (4c) de la pointe (4).

## Claims

1. A trocar intended for laparoscopy and for analogous operations on living beings, comprising an external hollow tube (1) that protects the tissues and the organs to be viewed and to be operated on, into which engages exactly a device (2) for perforating the anatomical membrane, the distal end of which is a polyhedral point characterized in that said perforating device (2) is formed by a hollow tube (3) the distal end of which is made up of cut away facets (4a) that permit either direct viewing of the path of the point (4) by means of an endoscope engaged for this purpose through the proximal part of said perforating device (2) or the passage of a point protection mechanism (5, 50) actuated coaxially and in an elastic way in the direction of said point (4) through at least one of the facets (4a) of the point.

2. A trocar according to the preceding Claim characterized in that the point protection mechanism (5, 50) is constituted by the assembly of a distal head (5a, 50a) in the shape of a warhead with several longitudinal sectors , the role of which is to slide along the sharp edges (4b) of the point (4), and an actuating assembly (5b) that elastically urges said distal head (5a, 50a) towards the front of the perforating device (2) in such a way as to release the point (4) only when sufficient recoil force is applied to said head (5a, 50a).

3. A trocar according to Claim 2, characterized in that the head (50a) of the point protection mechanism (50) has the shape of a staged warhead comprising at least one tapered end portion (52) and a second stage (54) of cross section slightly less than the internal section of the tube (3) of the trocar, linked to one another by a region (53) with the shape of a truncated cone.

4. A trocar according to Claim 3, characterized in that the section of the end portion (52) is the least possible that does not risk damaging the organs after the start of the perforation, the length of said portion (52) being sufficient so that, when the end 4c of the point 4 is just covered by said portion (52), the sharp cutting edges (4b) of the point (4) remain protruding sufficiently to finish the perforation.

5. A trocar according to any one of Claims 3 or 4, characterized in that the linking region (53) has an angle of inclination with respect to the longitudinal axis of the point protection mechanism (50) that is identical to the angle of inclination of the sharp edges 4b of the point 4.

6. A trocar according to any one of the preceding Claims, characterized in that the respective lengths of the point protection mechanism (5, 50) and of the hollow tube (3) of the perforation device (2) are such that in the at-rest position, the distal head (5a, 50a) of said mechanism (5, 50) completely surrounds the point (4).

7. A trocar according to the preceding Claim, characterized in that the rounded distal end of the warhead formed by said head (5a, 50a) goes a few millimeters past the extremity (4c) of the point (4).

## Patentansprüche

1. Trokar für die Koelioskopie und ähnliche Operationen an Lebewesen, mit einer externen hohlen Röhre (1), die die zu visualisierenden oder zu operierenden Gewebe und Organe schützt, in die sich eine Lochvorrichtung (2) der anatomischen Membran schlüssig einfügt, deren distales Ende eine vieleckige Spitze ist, dadurch gekennzeichnet, dass die besagte Lochvorrichtung (2) aus einer hohlen Röhre (3) besteht, deren distales Ende aus ausgehöhlten Facetten (4a) besteht, um entweder eine direkte Visualisierung des Weges der Spitze (4) über ein Endoskop zu ermöglichen, das zu diesem Zweck mit dem proximalen Teil der besagten Lochvorrichtung (2) eingeführt wird, oder um den Durchgang eines Spitzenschutzmechanismusses (5, 50) zu ermöglichen, der koaxial und elastisch in Richtung der besagten Spitze (4) betätigt wird, über mindestens eine der Facetten (4a) derselben.

2. Trokar nach dem vorstehend genannten Anspruch, daduch gekennzeichnet, dass der Spitzenschutzmechanismus (5, 50) aus der Zusammensetzung eines distalen, granatenförmigen Kopfes (5a, 50a) mit mehreren Längsabschnitten besteht, deren Aufgabe es ist, entlang den Kanten (4b) der Spitze (4) zu gleiten, und aus einer Betätigungsbaugruppe (5b), die den besagten distalen Kopf (5a, 50a) vorne an der Lochvorrichtung (2) elastisch beaufschlagt, so dass die Spitze (4) erst dann freigesetzt wird, wenn auf den besagten Kopf (5a, 50a) eine ausreichende Rückstellkraft ausgeübt wird.

3. Trokar nach Anspruch 2, dadurch gekennzeichnet, dass der Kopf (50a) des Spitzenschutzmechanismusses (50) die Form einer Granate mit mehreren Etagen aufweist, mit mindestens einem zugespitzten Endteil (52) und mit einer zweiten Etage (54) mit einem etwas kleineren Querschnitt als der Innenquerschnitt der Röhre (3) des Trokars, die über eine kegelstupfförmige Zone (53) miteinander verbunden sind.

4. Trokar nach Anspruch 3, dadurch gekennzeichnet, dass der Querschnitt des Endteils (52) so klein wie möglich ist, um die Organe nach dem Beginn des Lochens nicht zu verletzen, wobei die Länge des besagten Teiles (52) ausreichend ist, damit, wenn das Ende 4c der Spitze 4 gerade vom besagten Teil (52) überdeckt wird, die Schneidkanten (4b) der Spitze (4) genügend ausgekragt bleiben, um das Lochen zu beenden.

5. Trokar nach einem der Ansprüche 3 oder 4, dadurch gekennzeichnet, dass die Anschlusszone (53) in Bezug auf die Längsachse des Spitzenschutzmechanismusses (50) eine Neigung aufweist, die mit der Neigung der Kanten 4b der Spitze 4 identisch ist.

6. Trokar nach einem der vorstehend genannten Ansprüche, dadurch gekennzeichnet, dass die jeweiligen Längen des Spitzenschutzmechanismusses (5, 50) und der hohlen Röhre (3) der Lochvorrichtung (2) so vorgesehen sind, dass der distale Kopf (5a, 50a) des besagten Mechanismusses (5, 50) in Ruhestellung die Spitze (4) völlig umgibt.

7. Trokar nach dem vorstehend genannten Anspruch, dadurch gekennzeichnet, dass das distale abgerundete Ende der vom besagten Kopf (5a, 50a) gebildeten Granate das Ende (4c) der Spitze (4) um einige Millimeter überschreitet.
